# EUROPEAN PATENT SPECIFICATION

(11) **EP 1 590 046 B1**
(45) Date of publication and mention of the grant of the patent: **31.10.2018**
(21) Application number: 03786164.8
(22) Date of filing: 30.12.2003
(51) Int. Cl.: A61P 31/18, A61P 3/02, A61K 45/06, A61K 31/07, A61K 31/51, A61K 31/525, A61K 31/197, A61K 31/4415, A61K 31/714, A61K 31/375, A61K 31/592, A61K 31/593, A61K 31/355, A61K 31/122, A61K 31/519, A61K 33/04, A61K 33/06, A61K 33/24, A61K 33/26

(54) **COMPOSITION FOR THE TREATMENT OF HIV OR AIDS**
PHARMAZEUTISCHE ZUSAMMENSETZUNG ZUR BEHANDLUNG VON HIV-INFEKTIONEN UND AIDS
COMPOSITION POUR LE TRAITEMENT DU VIH OU DU SIDA

(30) Priority: 03.01.2003 GB 0300078
(43) Date of publication of application: 02.11.2005
(73) Proprietor: VITABIOTICS LIMITED, London NW2 7HF (GB)
(72) Inventor: TAYLOR, Robert, London W9 1AH (GB); LALVANI, Kartar, London NW2 7HF (GB)
(86) International application number: PCT/GB2003/005688
(87) International publication number: WO 2004/060487

(56) References cited:
- EP-A- 0 960 572
- OGUNTIBEJU O O ET AL: "Importance of vitamin and mineral supplementation in HIV/AIDS patients to improve their nutritional and immunological status" PAKISTAN JOURNAL OF MEDICAL SCIENCES 2003 PAKISTAN, vol. 19, no. 3, 2003, pages 217-219, XP0000902700 ISSN: 1682-024X
- BATTERHAM M ET AL: "A preliminary open label dose comparison using an antioxidant regimen to determine the effect on viral load and oxidative stress in men with HIV/AIDS" EUROPEAN JOURNAL OF CLINICAL NUTRITION, vol. 55, no. 2, February 2001 (2001-02), pages 107-114, XP0009026998 & ISSN: 0954-3007
- GARLAND MIRIAM ET AL: "Antioxidants and progression of human immunodeficiency virus (HIV) disease" NUTRITION RESEARCH, vol. 19, no. 8, August 1999 (1999-08), pages 1259-1276, XP0001179955 & ISSN: 0271-5317
- KELLY PAUL ET AL: "Micronutrient supplementation in the AIDS diarrhoea-wasting syndrome in Zambia: A randomized controlled trial" AIDS (HAGERSTOWN), vol. 13, no. 4, 11 March 1999 (1999-03-11), pages 495-500, XP0009027004 & ISSN: 0269-9370
- CONSTANS J ET AL: "Oxidative stress and HIV infection: Precisions on a concept and a new therapeutic approach" ANNALES DE MEDECINE INTERNE, vol. 146, no. 7, 1995, pages 514-520, XP0009026994 & ISSN: 0003-410X
- CHAN G ET AL: "Antioxidants may still have a role in HIV treatment." GMHC TREATMENT ISSUES : THE GAY MEN'S HEALTH CRISIS NEWSLETTER OF EXPERIMENTAL AIDS THERAPIES. SEP 1997, vol. 11, no. 9, September 1997 (1997-09), pages 6-12, XP0001179956 ISSN: 1077-1824
- SPRIETSMA J E: "MODERN DIETS AND DISEASE: NO-ZINC BALANCE UNDER TH1, ZINC AND NITROGEN MONOXIDE (NO) COLLECTIVELY PROTECT AGAINST VIRUSES, AIDS, AUTOIMMUNITY, DIABETES, ALLERGIES, ASTHMA, INFECTIOUS DISEASE, ATHEROSCLEROSIS AND CANCER" MEDICAL HYPOTHESES, EDEN PRESS, PENRITH, US, vol. 53, no. 1, 1999, pages 6-16, XP000972420 ISSN: 0306-9877
- MALVY D ET AL: "Essential micronutrients and HIV infection: Acknowledged review and interventional perspectives" NUTRITION CLINIQUE ET METABOLISME 1998 FRANCE, vol. 12, no. 3, 1998, pages 187-197, XP0009027001 ISSN: 0985-0562
- JARIWALLA R J: "MICRO-NUTRIENT IMBALANCE IN HIV INFECTION AND AIDS: RELEVANCE TO PATHOGENESIS AND THERAPY" JOURNAL OF NUTRITIONAL MEDICINE, CARFAX PUBLISHING, ABINGTON, GB, vol. 5, no. 3, 1995, pages 297-306, XP002058716 ISSN: 0955-6664
- COODLEY G O ET AL: "Micronutrients in HIV-infected women" JOURNAL OF WOMEN'S HEALTH 1995 UNITED STATES, vol. 4, no. 3, 1995, pages 303-311, XP0009027003 ISSN: 1059-7115
- SEMBA R D ET AL: "Micronutrients and the pathogenesis of human immunodeficiency virus infection" BRITISH JOURNAL OF NUTRITION, vol. 81, no. 3, March 1999 (1999-03), pages 181-189, XP0009026999 & ISSN: 0007-1145
- SKURNICK JOAN H ET AL: "Micronutrient profiles in HIV-1-infected heterosexual adults" JOURNAL OF ACQUIRED IMMUNE DEFICIENCY SYNDROMES AND HUMAN RETROVIROLOGY, vol. 12, no. 1, 1996, pages 75-83, XP0009026993 & ISSN: 1077-9450
- MAHMOOD N ET AL: "INHIBITION OF HIV INFECTION BY FLAVANOIDS" ANTIVIRAL RESEARCH, ELSEVIER SCIENCE BV., AMSTERDAM, NL, vol. 22, no. 2/3, 1993, pages 189-199, XP001039821 ISSN: 0166-3542

## Description

### TECHNICAL FIELD

This invention concerns a composition of biochemical constituents for use in the treatment of the HIV virus and AIDS.

### BACKGROUND ART

Over thirty three million people are currently living with HIV or AIDS. The vast majority of these are in the developing world. A number of observational studies have reported an association between low micro nutrient levels, accelerated 111V-1 disease progression and increased HIV-1 shedding in vaginal secretions. Only two randomised intervention trials of micro nutrient supplementation in HIV-1 infection have been conducted to date, one in pregnant women in Tanzania, and the other in hospital patients in Canada, and both showed benefit. These reports raise the possibility that micro nutrient supplementation may retard HIV-1 disease progression and reduce sexual transmission of the virus.

Evaluation of micro nutrient supplement therapies is of great importance for the developing world where very few patients have access to antiretroviral or other therapies and where increasing the micro nutrient intake of populations is likely to be a feasible and cost-effective public health measure.

The treatment recommended by state hospitals for HIV-infected patients is triple antiretroviral drug therapy including a protease inhibitor. In reality, not all patients receive AZT monotherapy, and almost none receive double or triple drug therapy. The resources available for drugs have diminished still further during the current economic crisis (Science [1998] 280: 1873, AIDS [1998] 12 : 1521, AIDS [1992] 6 : 393). Highly active antiretroviral combination therapy is unaffordable for the overwhelming majority, and so cheap effective alternative therapies are required urgently. There is increasing evidence that vitamin and mineral deficiencies may play an important role in HIV disease progression and transmission. In principle there are several reasons why this might be so.

Firstly, HIV patients are under oxidative stress from infection and progressive loss of CD4 T-cells (Free Radical Biology and Medicine [1996] 5 : 641). Secondly, a number of micro nutrients are required for normal immune function and are thus important for fighting infection (Nutrition [1998] 14 : 634, Nutrition Reviews [1998] 56 : S38, Clinical Infectious Diseases [1998] 26 : 711). Thirdly, overall nutritional intake is reduced since a number of HIV-associated clinical conditions including mucosal lesions of the oropharynx and oesophagus, fever and malignancies, decrease appetite while other HIV-associated conditions such as diarrhoea and malabsorption, increase demand for nutrients or impair their absorption (Gut [1997] 41 : 805, Journal of the American Dietary Association [1994] 94 : 1019, American Journal of clinical nutrition [1993] 58 : 417) .

Several observational studies have reported the role of micro nutrient levels in HIV-infection in developed countries. Low plasma levels of vitamin A (Archives of internal medicine [1993] 153 : 2149, Journal of infectious diseases [1995] 171 : 1196), Vitamin B12 [Journal of nutrition [1997] 127 : 345), selenium (Journal of acquired immune deficiency syndrome Hum. Retrovirl. [1997] 15 : 370) and zinc [AIDS [1995] 9 : 1051) have all been described as risk factors for mortality or faster progression to AIDS during HIV-infection.

Further evidence of the role of micro nutrient levels in HIV-infection are provided by a cohort study among 280 HIV-1 infected homosexual men followed for 8 years, conducted in the USA. This study found that high intakes of B1, B2, B6 and niacin, as assessed by a self administered food frequency questionnaire, were associated with reduced progression to AIDS and improved survival (American journal of journal epidemiology [1993] 138 : 937, American journal of epidemiology [1996] 143: 1244) but the relationship between vitamin A and progression to AIDS was U-shaped with an intake between 2 to 4 times the recommended dietary intake being associated with reduced progression and improved survival, and any increase of zinc intake was associated with poorer survival.

However, in all of these studies the use of plasma vitamin or mineral levels to assess status is problematic. Plasma levels are frequently too insensitive or nonspecific to be useful tools for assessing micro nutrient deficiencies and this is particularly the case during infection. The only effective way to disentangle cause and effect in studies of micro nutrients and slower rate of disease progression is to conduct intervention trials.

An important factor in the sexual transmission of HIV infection is the level of shedding of HIV-infected cells. A recent cross-sectional study conducted in Kenya has shown that low plasma vitamin A was associated with an up to 13-fold increased risk of HIV-1 DNA vaginal shedding, after controlling for CD4 count (Lancet [1997] 350 : 922). Little is known of the association between micro nutrient deficiency and secretions of HIV-infected cells in semen. However, a recent study from Malawi has shown that seminal HIV-1 RNA concentrations were, on average, 8 times higher among men with urethritis than among men without urethritis (Lancet [1997] 349 : 1868), and it has been suggested that African HIV-infected men have higher levels of HIV in blood and semen than patients in developed countries at similar levels of CD4 count (Journal of infectious diseases [1998] 177: 1742). Furthermore, a study conducted at Siriraj Hospital has also shown a high rate of HIV seminal and cervico-vaginal shedding, raising the possibility that this may, in part, have promoted the rapid spread of subtype-E by heterosexual transmission in Thailand (Journal of medical association of Thailand [1997] 80 : 348) .

Two randomised controlled trials provide support for micro nutrient supplementation in HIV-infection. In the first, 1075 HIV-infected pregnant women in Tanzania, with mean CD4 cell count at baseline of 449 cells/mL, were randomised to receive a daily dose of either vitamin A alone, multivitamins excluding vitamin A, multivitamins and vitamin A combined, or placebo

(Epidemiology [1998] 9 : 457), from 12 to 27 weeks of gestation to the time of delivery. At 6 and 30 weeks postpartum, the average increase in CD4 count, respectively, was 33% and 40% higher among women who had received multivitamins than among women who had not received multivitamins.

In a second randomised controlled trial, 49 Canadian adult HIV-infected patients, 24 of whom had AIDS, were randomised to receive either 800 IU of vitamin E combined with 1000 mg of vitamin C or a placebo, daily for 3-months (AIDS [1998] 12 : 1653). Patients in the two groups were comparable at baseline but by the end of the study period, average viral load was approximately 1.0 logio copies /ml lower in the vitamin group than in the placebo group (p=0.1), a figure that is considered to provide clinical benefit (Science [1996] 272: 1167). Furthermore, the intervention reduced oxidative stress. The effect on CD4 count was not reported.

There are differing potential mechanisms for a protective effect from micro nutrient supplements (Epidemiology [1998] 9 : 457). In brief, a protective effect of micro nutrients is likely to be mediated through enhancing the immune system by enhanced proliferation and maturation of immune cells, and improved function of natural killer cells and T-cell subsets (including CD4). This in turn might reduce the risk of opportunistic infections and possibly reduce viral load and virus shedding. In non-HIV-infected individuals, there is considerable evidence, some from clinical trials (Lancet [1992] 340 : 1124), that micro nutrient supplements enhance cellular and humoral immunity. Studies in HIV -infection have also shown that retinoic acid, carotenoid halocynthiaxanthin, and vitamin E might block the replication of HIV in vitro.

Much of the data have been reported from observational studies, and suffer the interpretation problems discussed above and from the possibility that the associations were merely a marker for disease stage or the result of the confounding effect of some other unknown factor. Convincing evidence of the potential role of micro nutrients can only be provided by randomised controlled trials, of which, as discussed above, two have been conducted; but one was in pregnant women and did not measure any clinical endpoints in the mother, while the other was a preliminary study that was too small in size. Thus the clinical relevance of these findings is not clear. Furthermore, it is not known whether the findings of those studies which were only carried out on pregnant women can be generalised to other populations such as men, STD attendees or sex-workers.

Trials are currently underway in several African countries to determine whether vitamin A might affect vertical transmission of HIV (Proceedings of the Nutrition Society 119981 57 : 159). However, the fact that multiple micro nutrient deficiencies usually co-exist and often interact might make it difficult to interpret and generalize the results from single micro nutrient studies. For example, zinc is required for the mobilisation of vitamin A from liver stores, and thus the benefits of vitamin A would be missed in a zinc-deficient population if this was not given alongside zinc. Furthermore, since several micro nutrients are required for the same immune functions, repletion of the limiting micro nutrient may only increase the function up to a level where another nutrient becomes limiting. Thus, it does not seem reasonable to evaluate a single micro nutrient in the context of multiple co-existing and interacting deficiencies.

### DISCLOSURE OF INVENTION

The problem with many of the studies carried out on HIV and AIDS is that they do not account for all of the potential biochemical needs of the patients. This invention has therefore been devised to provide a comprehensive formula for the immune system, with effective levels of antioxidants and a strong emphasis on the vital mineral selenium. Vitamins C and E are included because they circulate in the body to help protect against the damage caused by free radicals. Other nutrients such as betacarotene are included because they protect DNA and cells from within. The formulation has also been devised to provide those minerals vital in assisting the body's antioxidant enzymes Superoxide Dismutase, Catalase, and Glutathione Peroxidase. These eliminate harmful chemicals, including free radicals and peroxides. The action of these enzymes are dependent upon the presence of manganese, copper, zinc, iron and selenium. Amino acids are needed by the body's antioxidant enzymes. People with HIV or AIDS are normally, due to reduced appetite, lacking in other vital nutrients, such as vitamin B1, iodine, chromium and manganese. Although these are not essential for the immune system, their deficiency will normally occur as a result of a deficency of the immune system, and for that reason are included in the formulation. This formulation could therefore also have effect other than by effecting CD4 cell numbers or activity. This medical aim of the invention is therefore to provide regular micro nutrient supplementation on HIV-1 progression,on seminal HIV-RNA secretion in men and on HIV-1 RNA shedding in endocervical and vaginal secretions in women. The purpose of the formulation described is the treatment of the HIV virus and the AIDS to which it usually leads. The primary interest is in developing countries, which are of major public health importance. There are no recommended daily allowances of micro nutrients for people with infections, but it is thought that their requirements are considerably higher than those of healthy people (Nutrition [1994] 10 : 16, Journal of acquired immune deficiency syndrome [1995] 8 : 199). These doses generally exceed the recommended daily allowance for healthy people, but are similar to those used in the randomised trials conducted in Canada and Kenya (AIDS [1998] 12 : 1653, Lancet [1998] 351 : 1477). The dosages used in the formulation are purposefully higher than would be used in healthy people, in order to account for the specific needs of the medical disorders for which the formulation is intended.

European Journal of Clinical Nutrition [2001] 55 (2) 107-114 (M.Batterham et al), which was referred to as D3, is the closest prior art. It contains some of the constituents of the present invention. Although it concerns HIV/AIDS, it does not affect the death rate and was only able to improve markers of antioxidant defence and oxidative stress.
The optimal daily dosage to be used in the formulation is the following :

| | | |
|---|---|---|
| Vitamin A | 1500 mcg | (5000 I.U.) |
| Betacarotene | 6 mg | |
| Thiamin (vitamin B1) | 15 mg | |
| Riboflavin (vitamin B2) | 10 mg | |
| Panthothenic Acid (vitamin B5) | 20 mg | |
| Vitamin B6 | 20 mg | |
| Vitamin B12 | 15 mcg | |
| Folacin (folic acid) | 500 mcg | |
| Vitamin C | 300 mg | |
| Vitamin D | 10 mcg | (400 I.U.) |
| Vitamin E (natural source) | 120 mg | |
| Vitamin K | 95 mcg | |
| Iron | 6 m g | |
| Magnesium | 100 mg | |
| Zinc | 15 mg | |
| Iodine | 200 mcg | |
| Copper | 2 mg | |
| Manganese | 4 mg | |
| Selenium | 200 mcg | |
| Chromium | 100 mcg | |
| Cystine | 40 mg | |
| Methionine | 40 mg | |
| Bioflavonoids | 30 mg | |

### INDUSTRIAL APPLICABILITY

Therefore, according to this invention, there is a pharmaceutical composition, or the use of that composition in the manufacture of a medicament, or a method of treatment including the use of that pharmaceutical composition for combined, sequential or simultaneous administration, in any form, via any route, for the treatment of the the HIV virus or AIDS. The primary constituents of that formulation are Vitamin A, Vitamin C, Vitamin E, and at least 20mcg of Selenium. That formulation can also comprise Betacarotene, Vitamin B1, Vitamin B2, Vitamin B5, Vitamin B6, Vitamin B12, Folic acid, Vitamin D, Vitamin K, Iron, Magnesium, Zinc, Iodine, Copper, Manganese, Chromium, Cystine, Methionine and Bioflavonoids.

## Claims

1. A pharmaceutical composition for use in the treatment of HIV or AIDS, for combined, sequential or simultaneous administration, in any form, via any biological route, comprising vitamin A, beta-carotene, vitamin B1, vitamin B2, vitamin B5, vitamin B6, vitamin B12, folic acid, vitamin C, vitamin D, vitamin E, vitamin K, iron, magnesium, zinc, iodine, copper, manganese, selenium, chromium, cystine, methionine and bioflavonoids.

2. A pharmaceutical composition as claimed in Claim 1 for use as in Claim 1 wherein the pharmaceutical composition does not comprise more than 300 mg of amino acids in a protein or amino acid complex form.

3. A pharmaceutical composition as claimed in Claim 1 for use as in Claim 1 consisting essentially of the components defined in the relevant claim.

4. A pharmaceutical composition as claimed in any of the preceding claims for the treatment of AIDS.

5. A pharmaceutical composition as claimed in any of Claims 1 to 3 for the treatment of HIV.

6. A pharmaceutical composition as defined in any of Claims 1 to 5, for use as in Claim 1, wherein improvement in the medical condition is achieved through a mechanism that is independent of CD4 cell numbers or activity.

## Patentansprüche

1. Pharmazeutische Zusammensetzung zur Verwendung bei der Behandlung von HIV oder AIDS zur kombinierten, aufeinanderfolgenden oder gleichzeitigen Verabreichung in beliebiger Form über eine beliebige biologische Route, umfassend Vitamin A, beta-Carotin, Vitamin B1, Vitamin B2, Vitamin B5, Vitamin B6, Vitamin B12, Folsäure, Vitamin C, Vitamin D, Vitamin E, Vitamin K, Eisen, Magnesium, Zink, Iod, Kupfer, Mangan, Selen, Chrom, Cystin, Methionin und Bioflavonoide.

2. Pharmazeutische Zusammensetzung nach Anspruch 1 zur Verwendung nach Anspruch 1, wobei die pharmazeutische Zusammensetzung nicht mehr als 300 mg Aminosäuren in einer Proteinen- oder Aminosäurekomplexform umfasst.

3. Pharmazeutische Zusammensetzung nach Anspruch 1 zur Verwendung nach Anspruch 1, welche im Wesentlichen aus den in dem betreffenden Anspruch definierten Komponenten besteht.

4. Pharmazeutische Zusammensetzung nach einem der vorhergehenden Ansprüche zur Behandlung von AIDS.

5. Pharmazeutische Zusammensetzung nach einem der Ansprüche 1 bis 3 zur Behandlung von HIV.

6. Pharmazeutische Zusammensetzung nach einem der Ansprüche 1 bis 5 zur Verwendung nach Anspruch 1, wobei die Verbesserung des medizinischen Zustands durch einen von der CD4-Zellzahl oder -Aktivität unabhängigen Mechanismus erzielt wird.

## Revendications

1. Composition pharmaceutique pour utilisation dans le traitement du VIH et du SIDA, pour administration combinée, séquentielle ou simultanée, sous n'importe quelle forme, via n'importe quelle voie biologique, comprenant les composants suivants : vitamine A, bêta-carotène, vitamine B1, vitamine B2, vitamine B5, vitamine B6, vitamine B12, acide folique, vitamine C, vitamine D, vitamine E, vitamine K, fer, magnésium, zinc, iode, cuivre, manganèse, sélénium, chrome, cystine, méthionine et bioflavonoïdes.

2. Composition pharmaceutique selon la Revendication 1 pour utilisation selon la Revendication 1, où la composition pharmaceutique ne comprend pas plus de 300 mg d'acides aminés sous forme de complexe d'acides aminés ou de protéines.

3. Composition pharmaceutique selon la Revendication 1 pour utilisation selon la Revendication 1 essentiellement constituée des composants définis dans la revendication correspondante.

4. Composition pharmaceutique selon l'une quelconque des revendications précédentes destinée au traitement du SIDA.

5. Composition pharmaceutique selon l'une quelconque des Revendications 1 à 3 destinée au traitement du VIH.

6. Composition pharmaceutique selon l'une quelconque des Revendications 1 à 5, pour utilisation selon la Revendication 1, où l'amélioration de l'état pathologique est obtenue via un mécanisme qui est indépendant du nombre ou de l'activité des cellules CD4.
